# FASCICULE DE BREVET EUROPEEN

(11) **EP 0 452 229 B1**
(45) Date de publication et mention de la délivrance du brevet: **03.08.1994**
(21) Numéro de dépôt: 91420101.7
(22) Date de dépôt: 27.03.1991
(51) Int. Cl.: A61M 1/16, A61M 1/34, B01D 61/26

(54) **Procédé et dispositif de préparation d'un liquide de substitution**
Vorrichtung und Verfahren zur Herstellung einer Substitutionsflüssigkeit
Procedure and device to prepare a substitution liquid

(30) Priorité: 13.04.1990 FR 9005034
(43) Date de publication de la demande: 16.10.1991
(73) Titulaire: HOSPAL INDUSTRIE, F-69883 Meyzieu Cédex (FR)
(72) Inventeur: Delaunay, Marc, F-69150 Decines (FR)

(56) Documents cités:
- EP-A- 0 097 863
- EP-A- 0 228 782
- EP-A- 0 276 376

## Description

La présente invention concerne un procédé et un dispositif de préparation d'un liquide de substitution.

Par liquide de substitution, on entend un liquide destiné à être injecté dans le circuit vasculaire d'un patient souffrant, par exemple, de brûlures graves ou ayant perdu du sang en quantité importante ou encore, d'un patient soumis à un traitement d'épuration sanguine par hémofiltration ou hémodiafiltration, prescrit à a suite de la perte provisoire ou définitive de ses fonctions rénales.

L'invention trouve un champ d'application particulièrement intéressant dans le cas d'un traitement de ce type, car la quantité de liquide de substitution nécessaire au cours d'une séance d'hémofiltration ou d'hémodiafiltration peut être très importante (jusqu'à trente litres, selon les patients, pour une séance de quatre heures).

Un liquide de substitution doit être stérile et apyrogène, c'est-à-dire être exempt de tout germe pathogène et de toute substance susceptible d'engendrer une réaction fébrile chez le patient. En outre, il doit être isotonique au sang, c'est-à-dire avoir la même concentration électrolytique que le sang. La proportion relative des cations et des anions résultant de la dissociation des électrolytes du liquide de substitution est ajustée en fonction des besoins du patient, selon qu'il est besoin de maintenir ou de rétablir son équilibre électrolytique. Pour finir, un liquide de substitution doit contenir au moins ceux des électrolytes du sang qui jouent un rôle essentiel dans le métabolisme, c'est-à-dire, dissociés sous forme de cations et d'anions, le sodium, le potassium, le calcium et le magnésium pour les cations, et les chlorures et les bicarbonates pour les anions, les bicarbonates assurant dans une mesure déterminante la fonction d'agent tampon.

Les caractéristiques d'un liquide de substitution qui viennent d'être rappelées, à savoir donc la stérilité, le caractère apyrogène, l'isotonie au sang et la composition électrolytique spécifique, font que la préparation d'un liquide de substitution est délicate et exige des précautions particulières.

Dans les hôpitaux, on utilise usuellement des liquides de substitution de composition standard, préparés industriellement par des fournisseurs spécialisés. Ces liquides présentent l'inconvénient d'être coûteux. En outre, lorsqu'ils contiennent des bicarbonates, leur conservation pose des problèmes mal maîtrisés actuellement, dus à la combinaison de deux facteurs : le premier de ces facteurs est que les bicarbonates sont instables et se décomposent spontanément et continuement en dioxyde de carbone lorsqu'ils ne sont pas confinés de façon étanche, et le second de ces facteurs est que les poches utilisables pour conditionner les liquides de substitution sont, en général, perméables au gaz. De la sorte, s'il apparaît souhaitable d'utiliser un liquide de substitution ayant une concentration précise en bicarbonate, il faut utiliser ce liquide très rapidement après son conditionnement, ce qui constitue une contrainte pour l'utilisateur.

Le brevet US 4 702 829 décrit un dispositif de préparation de liquide de substitution, prévu spécifiquement pour être utilisé lors d'un traitement par hémodiafiltration, qui pallie partiellement ces inconvénients. Dans ce brevet, on propose de préparer un liquide de substitution à partir d'un liquide de dialyse fabriqué dans un générateur de liquide de dialyse, les liquides de dialyse ayant généralement sensiblement la composition des liquides de substitution standard, mais n'étant ni stériles, ni apyrogènes. Le dispositif décrit dans ce brevet est une dérivation du circuit de dialyse qui comporte deux filtres stériles entre lesquels est disposée une pompe de circulation.

Outre qu'il ne permet de répondre qu'à un besoin très spécifique, ce dispositif présente les inconvénients inhérents à l'utilisation de filtres coûteux qui doivent être changés régulièrement et dont le colmatage éventuel doit toujours pouvoir être détecté à temps.

Un but de la présente invention est de proposer un procédé de préparation de liquide de substitution quine soit pas spécifique à un traitement particulier, qui soit simple et relativement peu coûteux à mettre en oeuvre.

Un autre but de l'invention est de réaliser un dispositif permettant de mettre en oeuvre ce procédé.

Un autre but de l'invention est de réaliser un tel dispositif qui soit particulièrement adapté à un traitement par hémodiafiltration.

Conformément à l'invention, pour atteindre ces buts, on prévoit un procédé de préparation d'un liquide de substitution, caractérisé en ce qu'il consiste à faire s'écouler, de part et d'autre d'une membrane semi-perméable d'un échangeur, un premier liquide stérile et apyrogène et un second liquide, ces liquides contenant au moins certains des électrolytes du sang dont au moins un agent tampon ou au moins un élément précurseur d'agent tampon, au moins certains de ces électrolytes ayant des concentrations différentes dans les deux liquides en amont de l'échangeur, le liquide de substitution étant constitué par le premier liquide en aval de l'échangeur.

Ce procédé présente l'avantage qu'il permet d'enrichir, sans risque de contamination, un premier liquide stérile et apyrogène avec des électrolytes déterminés et dans des proportions déterminées, au moyen d'un second liquide qui peut n'être ni stérile, ni apyrogène, le premier liquide étant avantageusement choisi parmi les solutions standard, de composition électrolytique simple, qui sont facilement disponibles et bon marché.

Selon une caractéristique de l'invention, le second liquide est utilisé, en aval de l'échangeur comme liquide de dialyse.

Cette disposition est particulièrement avantageuse, dans le cas de traitement par hémodiafiltration, car elle permet la préparation simultanée d'un liquide de substitution et d'un liquide de dialyse avec des moyens de préparation communs.

Selon une autre caractéristique de l'invention, l'un des liquides est chauffé en amont de l'échangeur de façon que le premier liquide ait, en aval de l'échangeur, une température voisine de celle du corps humain.

Lorsque le liquide chauffé est le second liquide, l'échangeur, qui est d'abord prévu pour permettre les transferts d'ions, est utilisé comme échangeur thermique. Cette disposition est particulièrement avantageuse lorsque la source de second liquide est un générateur de liquide de dialyse, de tels générateurs comprenant usuellement des moyens de chauffage de liquide.

Conformément à l'invention, on prévoit en outre un dispositif de préparation d'un liquide de substitution, caractérisé en ce qu'il comporte un échangeur comprenant deux chambres séparées par une membrane semi-perméable, une première chambre ayant une entrée connectée à une source d'un premier liquide stérile et apyrogène et une sortie destinée à être connectée à un réceptacle (patient ou réservoir de liquide extemporané), une seconde chambre ayant une entrée connectée à une source d'un second liquide et une sortie destinée à être connectée éventuellement à un dialyseur, ces liquides contenant au moins certains des électrolytes du sang dont au moins un agent tampon ou au moins un élément précurseur d'agent tampon, au moins certains de ces électrolytes ayant des concentrations différentes dans les deux liquides en amont de l'échangeur.

D'autres caractéristiques et avantages de l'invention apparaîtront à la lecture de la description qui suit, faite en relation aux dessins annexés sur lesquels :
La figure 1 est un schéma d'un dispositif de préparation de liquide de substitution selon l'invention ; et
La figure 2 est un schéma de la combinaison d'un dispositif d'hémodiafiltration et d'un dispositif de préparation de liquide de substitution selon l'invention.

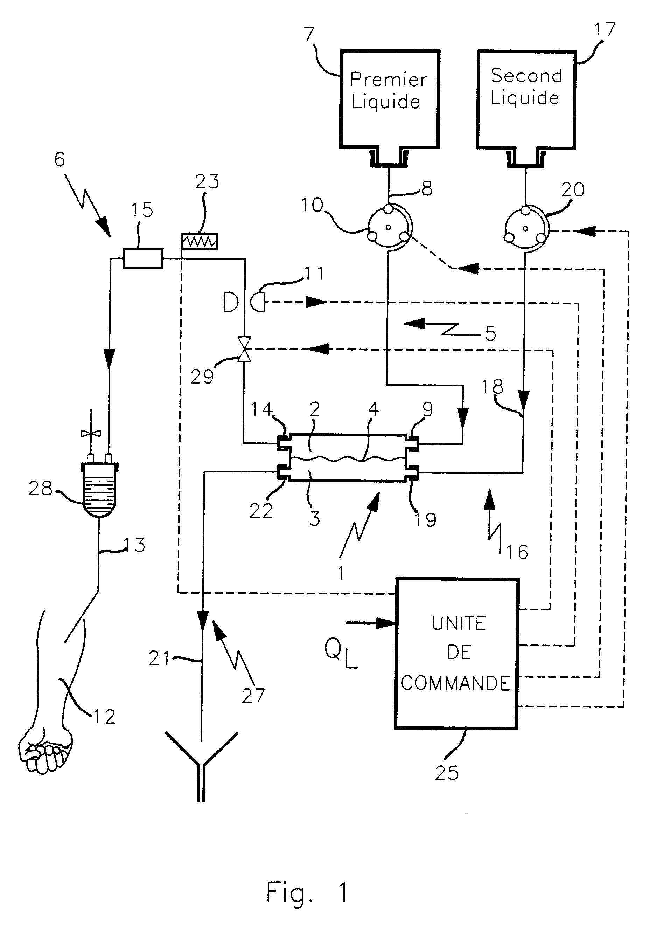

Le dispositif de préparation de liquide de substitution représenté sur la figure 1 comporte un échangeur 1 comprenant une première et une seconde chambres 2, 3 séparées par une membrane semi-perméable 4 et reliées respectivement à un premier et à un second circuits prévus pour l'écoulement de liquides dont la nature sera précisée plus loin.

Le premier circuit comprend deux parties 5, 6 situées respectivement en amont et en aval de l'échangeur 1, par rapport au sens de l'écoulement du liquide dans ce premier circuit. La partie amont 5 du premier circuit comprend un réservoir 7 pour un premier liquide, relié par une canalisation 8 à une entrée 9 de la première chambre 2 de l'échangeur 1. Une pompe péristaltique 10 est disposée sur cette partie du premier circuit pour provoquer l'écoulement du premier liquide du réservoir 7 vers l'échangeur 1 et en régler le débit. Un débitmètre 11 est disposé en aval de l'échangeur 1 pour mesurer précisément le débit du liquide injecté à un patient 12.

Dans une variante simplifiée du dispositif selon l'invention, le premier circuit ne comprend ni la pompe 10, ni le débitmètre 11 et l'écoulement du premier liquide est provoqué par gravité, le réservoir 7 étant placé en hauteur par rapport au patient 12. La canalisation 8 est alors munie d'une vanne pour le réglage du débit du premier liquide.

La partie aval 6 du premier circuit comporte une canalisation 13 ayant une extrémité raccordée à une sortie 14 de la première chambre 2 de l'échangeur 1 et comprenant à son autre extrémité une aiguille destinée à être enfoncée dans une veine du patient 12. Cette seconde extrémité peut aussi, le cas échéant, être connectée à un réservoir de liquide de substitution extemporané. La canalisation 13 passe dans une chambre de dégazage 28. Par mesure de sécurité, un filtre antibactérien 15 peut être placé sur la canalisation 13 en amont de la chambre de dégazage 14.

Un organe de chauffage 23 est disposé dans la partie aval 6 du premier circuit pour porter le liquide injecté au patient 12 à une température voisine de 37°C, lorsque le débit de ce liquide est supérieur à environ un litre/heure (en-dessous de ce débit, il est admis qu'on peut injecter à un patient un liquide à température ambiante).

Le second circuit comprend également deux parties 16, 27 situées respectivement en amont et en aval de l'échangeur 1, par rapport au sens de l'écoulement du liquide dans ce deuxième circuit. La partie amont 16 du second circuit comprend un réservoir 17 pour un second liquide, relié par une canalisation 18 à une entrée 19 de la seconde chambre 3 de l'échangeur 1. Une pompe péristaltique 20 est disposée sur cette partie du second circuit pour provoquer l'écoulement du second liquide du réservoir 17 vers l'échangeur 1 et pour en régler le débit.

Dans une variante simplifiée du dispositif selon l'invention, on ne prévoit pas de pompe dans la partie amont 16 du second circuit et l'écoulement du second liquide est provoqué par gravité, le réservoir 17 étant placé en hauteur par rapport au patient 12. La canalisation 18 est alors munie d'une vanne pour le réglage du débit du second liquide.

Dans une variante du dispositif selon l'invention, l'organe de chauffage 23 est disposé non plus dans la partie aval 6 du premier circuit, mais dans la partie amont 16 du second circuit, le premier liquide étant alors réchauffé par le second liquide dans l'échangeur 1.

La partie aval 27 du second circuit comporte une canalisation 21 ayant une extrémité raccordée à une sortie 22 de la seconde chambre 3 de l'échangeur 1, et débouchant, à son autre extrémité, dans un circuit d'évacuation d'eaux usées.

Comme on peut le remarquer sur la figure 1, les parties amont 5, 16 et aval 6, 27 des premier et second circuits sont raccordées à l'échangeur 1 de façon que les premier et second liquides y circulent à co-courant. Cette disposition, qui n'est pas la plus favorable aux échanges entre les deux liquides, qui seront précisés plus loin, présente cependant l'avantage que les gradients de pression hydraulique de part et d'autre de la membrane sont de même signe : de la sorte, même si la membrane 4 de l'échangeur 1 est une membrane à haute perméabilité, le risque qu'il se produise une ultrafiltration du second liquide dans le premier circuit se trouve réduit.

Pour éliminer ce risque, inacceptable dans la mesure où le premier liquide ne doit être pollué en aucun cas, une perte de charge 29 est placée sur le premier circuit, en aval de l'échangeur 1, de façon à maintenir en permanence une pression transmembranaire positive entre la chambre 2 et la chambre 3 de l'échangeur 1. Cette perte de charge peut être réglable et être pilotée par une unité de commande 25, en fonction des pressions régnant respectivement dans les premier et second circuits au niveau de l'échangeur 1, pour maintenir sensiblement constante la pression transmembranaire.

L'unité de commande 25 sert en outre à piloter la pompe 10 en fonction d'un débit QL de liquide de substitution déterminé par le clinicien et de la mesure du débit du premier liquide fournie par le débitmètre 11, de façon que le débit du liquide de substitution injecté au patient 12 soit sensiblement égal à QL, compte tenu d'une ultrafiltration éventuelle dans l'échangeur 1 (laquelle dépend de la nature de la membrane 4). L'unité de commande 25 règle aussi l'intensité du chauffage produit par l'unité de chauffage 23, en fonction du débit du liquide de substitution. Dans certaines conditions d'utilisation de ce premier dispositif, définies par le rapport des débits dans l'échangeur 1 et par les caractéristiques de l'échangeur 1 (nature et surface de la membrane 4), il peut être intéressant aussi de prévoir que l'unité de commande 25 règle le débit de la pompe 20 en fonction du débit de la pompe 10, si on souhaite contrôler de façon précise les transferts ioniques dans l'échangeur 1, tels qu'ils sont précisés plus loin.

Le fonctionnement du dispositif qui vient d'être décrit repose sur le principe selon lequel deux liquides contenant des électrolytes dans des concentrations différentes échangent des ions lorsqu'ils sont mis en contact par l'intermédiaire d'une membrane semi-perméable, chaque type d'ion migrant par diffusion au travers de la membrane, du côté de la membrane où sa concentration est la plus forte vers le côté de la membrane où sa concentration est la plus faible, et ce jusqu'à l'équilibre des concentrations de part et d'autre de la membrane.

Il est important de noter que selon les débits respectifs des premier et second liquides dans l'échangeur 1 et selon les caractéristiques de l'échangeur 1 (nature et surface de la membrane 4) le taux de transfert ionique dans l'échangeur, dont la constance garantit la préparation d'un liquide de substitution de concentration électrolytique déterminée, dépend dans une mesure variable du rapport des débits dans l'échangeur. Dans la mesure du possible, compte tenu de la gamme des débits QL usuellement prescrits, on choisira avantageusement l'échangeur 1 et le débit du second liquide de façon à se placer dans des conditions où le taux de transfert ionique n'est pas ou peu influencé par une variation du débit du premier liquide. Si on ne peut pas s'y placer, on asservira alors la pompe 20 à la pompe 10, de façon que le rapport de leur débit respectif soit sensiblement constant, conformément à la disposition évoquée plus haut.

Conformément à l'invention, le premier liquide est une solution stérile et apyrogène ayant de préférence une composition électrolytique simple. Le second liquide est une solution qui peut n'être ni stérile, ni apyrogène et dont la composition électrolytique est déterminée en fonction de la composition électrolytique du premier liquide, du débit des liquides dans les premier et second circuits et des besoins spécifiques du patient 12, de façon que le liquide de substitution proprement dit, qui est le liquide qui s'écoule dans la partie aval 6 du premier circuit, c'est-à-dire le premier liquide enrichi en ions et, le cas échéant, réchauffé, par le second liquide, ait la composition électrolytique, la concentration ionique et la température appropriées au patient.

En principe, on prévoit d'utiliser un premier liquide de composition standard, facilement disponible sur le marché. Le second liquide, dont la préparation n'exige pas de précautions particulières et relève de la pratique pharmaceutique courante dans les hôpitaux, résulte avantageusement de la dilution de solutions salines concentrées ou de sels à l'état solide dans de l'eau déminéralisée.

A titre d'exemple, les trois couples de liquides suivants peuvent être utilisés dans le dispositif selon l'invention :

### Exemple 1 :

Le premier liquide est une solution de chlorure de sodium ayant une concentration sensiblement identique à celle du sang. Le second liquide contient les principaux électrolytes du sang, c'est-à-dire, dissociés sous forme de cations et d'anions, le sodium, le potassium, le calcium et le magnésium pour les cations, et les chlorures et les bicarbonates pour les anions, les bicarbonates assurant dans une mesure déterminante la fonction d'agent tampon (dans la suite, on englobera es bicarbonates sous un terme générique singulier, et on parlera du bicarbonate). Au lieu de cet agent tampon, le second liquide peut contenir des éléments précurseurs d'agent tampon, tels que de l'acétate ou de l'acétate additionné de lactate, qui sont transformés en bicarbonate par l'organisme. Si le second liquide contient du bicarbonate, on lui adjoint de l'acide acétique en quantité suffisante pour empêcher la précipitation des ions bicarbonate par les ions calcium et magnésium. La concentration du second liquide en ions sodium et en ions chlorure est identique à celle du premier liquide, de sorte qu'il ne se produit pas de diffusion sensible de ces ions à travers la membrane 4.

Avec ces deux liquides, les transferts diffusifs dans l'échangeur se font essentiellement du second liquide vers le premier liquide.

### Exemple 2 :

Le premier liquide est une solution de chlorures de sodium, de calcium et de magnésium. Le second liquide contient les principaux électrolytes du sang, dont le bicarbonate comme agent tampon, à l'exception des ions calcium et magnésium, de sorte que le problème posé par la précipitation indésirable des ions bicarbonates par les ions calcium et magnésium dans le second liquide ne se pose pas. Comme la concentration des ions calcium et magnésium dans le second liquide est nulle, il se produit un transfert diffusif de ces ions dans l'échangeur, du premier liquide vers le second liquide. Pour une concentration souhaitée des ions calcium et magnésium dans le liquide de substitution, il faut donc choisir un premier liquide dont la concentration en ions calcium et magnésium soit supérieure à cette concentration souhaitée, afin de compenser les pertes diffusives.

### Exemple 3 :

Le premier liquide est une solution de bicarbonate de sodium. Le second liquide contient les principaux électrolytes lu sang, à l'exception de bicarbonate. De façon similaire à ce qu'on observe dans le cas de l'exemple 2, comme la concentration des ions bicarbonate dans le second liquide est nulle, il se produit un transfert diffusif de ces ions dans l'échangeur, du premier liquide vers le second liquide. Pour une concentration souhaitée de bicarbonate dans le liquide de substitution, il faut donc choisir un premier liquide dont la concentration en bicarbonate soit supérieure à cette concentration souhaitée, afin de compenser les pertes diffusives.

Le fonctionnement du dispositif de préparation d'un liquide de substitution qui vient d'être décrit est le suivant. Les premier et second liquides ayant été respectivement choisi et préparé on met en place les réservoirs 7 et 17 et l'échangeur 1 sur un support (non représenté) du dispositif. De même, on installe les premier et second circuits en connectant les parties amont et aval 5, 16 et 6, 27 de ces circuits à l'échangeur 1 et aux réservoirs 7, 17. Avant de connecter la canalisation 8 du premier circuit au réservoir 7 de premier liquide, on rince le premier circuit et la première chambre 2 de l'échangeur 1 à laquelle il est relié avec un liquide stérile et apyrogène, puis on effectue le remplissage initial (opération généralement dite de "priming") du premier circuit et de la chambre 2 avec ce même liquide. Avantageusement on utilise le premier liquide comme liquide de rinçage et de remplissage initial.

Le débit souhaité QL du liquide de substitution ayant été introduit dans l'unité de commande 25, celle-ci commande la rotation de la pompe 10 de façon que le débit de la pompe 10 soit sensiblement égal à QL. Si on a prévu un asservissement de la pompe 20 à la pompe 10, l'unité de commande 25 commande en outre la rotation de la pompe 20 de façon que le taux d'échange dans l'échangeur soit sensiblement constant. Lorsque le liquide de remplissage initial a été totalement chassé du premier circuit par le premier liquide et que le second liquide a rempli complètement le second circuit et commence à s'écouler hors de la canalisation 21, le liquide qui sort de l'aiguille connectée à l'extrémité du premier circuit est le liquide de substitution désiré. L'aiguille peut alors être enfoncée dans la veine du patient 12 et l'injection commencer. A partir de l'information fournie par le débitmètre 11, qu'elle compare au débit souhaité QL du liquide de substitution, l'unité de commande 25 règle la rotation de la pompe 10 pour que le débit réel soit égal au débit souhaité.L'unité de commande 25 commande en outre la mise en marche de l'organe de chauffage 23 et en règle l'intensité en fonction de QL.

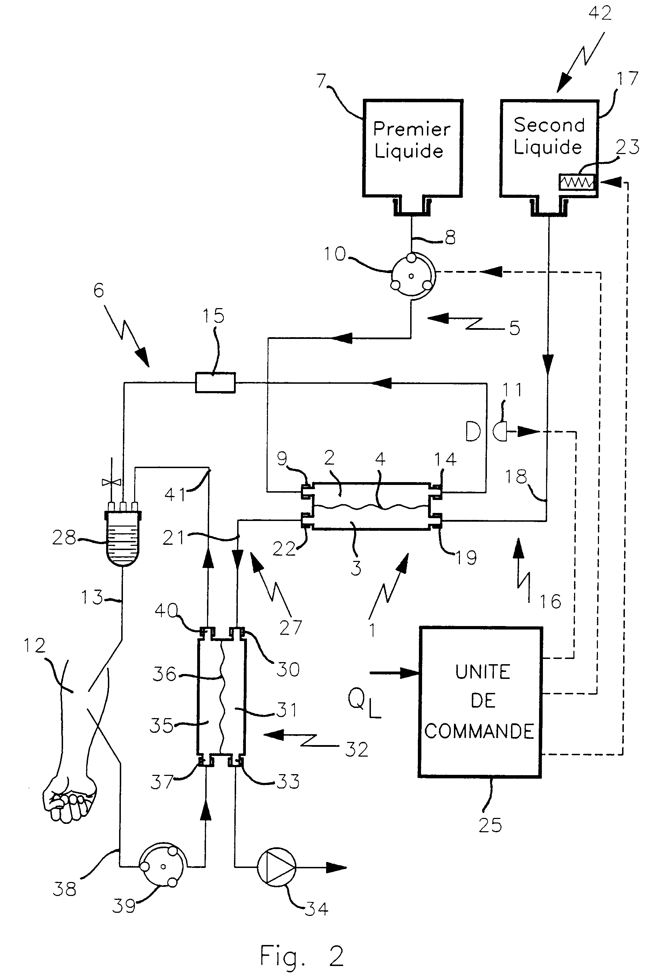

La figure 2 représente un second dispositif de préparation de liquide de substitution, qui est associé à un dispositif d'hémodiafiltration. Sur cette figure, les chiffres de référence utilisés sur la figure 1 ont été repris pour désigner les éléments correspondants de ce second dispositif selon l'invention.

Ce dispositif de préparation de liquide de substitution diffère du précédent essentiellement en ce que la partie aval 27 du second circuit, au lieu de déboucher à son extrémité libre dans un système d'évacuation d'eaux usées, est raccordée à une entrée 30 d'une première chambre 31 d'un dialyseur 32, dont la sortie 33 est connectée à un circuit de dialyse classique (partiellement représenté) sur lequel est disposé notamment une pompe 34 de circulation de liquide de dialyse. Dans le dispositif d'hémodiafiltration représenté sur la figure 2, c'est donc le liquide provenant du réservoir 17 de second liquide, qui, après être passé dans l'échangeur 1, sert de liquide de dialyse.

La seconde chambre 35 du dialyseur 32, qui est séparée de la première chambre 31 par une membrane semi-perméable 36, a une entrée 37 reliée à une ligne artérielle 38 sur laquelle est disposée une pompe 39 de circulation du sang, et une sortie 40 reliée à une ligne veineuse 41 débouchant dans la chambre de dégazage 28.

Dans le mode de réalisation de ce second dispositif de préparation de liquide de substitution représenté sur la figure 2 le second circuit ne comporte pas de pompe pour le second liquide, l'écoulement de celui-ci étant provoqué par la pompe 34 du dispositif d'hémodiafiltration.

Les pompes 10 et 34 fonctionnent indépendamment l'une de l'autre. Une caractéristique intéressante de ce dispositif réside dans le fait que, eu égard aux débits de liquide de dialyse et de liquide de substitution habituellement utilisés, il existe des échangeurs ayant des caractéristiques telles qu'une variation du débit de liquide de substitution, même dans une plage de débit assez large, n'a pas, pour un débit de liquide de dialyse donné, d'influence sur le taux de transfert ionique dans l'échangeur.

En d'autres termes, à partir de liquides de compositions respectives déterminées en amont de l'échangeur 1, pour un débit de liquide de dialyse donné, les compositions respectives des liquides en aval de l'échangeur 1 (liquide de dialyse, liquide de substitution) ne changeront pas sensiblement quel que soit le débit du liquide de substitution choisi dans une plage de débits donnée, assez large. A titre d'exemple, dans un échangeur équipé d'une membrane de environ 0,5 m², du type connu sous la dénomination commerciale CUPROPHAN, pour un débit de liquide de dialyse sensiblement égal à 500 ml/mn, l'équilibre entre les liquides dans l'échangeur 1 se fait quasiment à 100 % lorsque le débit du liquide de substitution est choisi dans une plage s'étendant de 0 à environ 50 ml/mn.

Par rapport au dispositif décrit en référence à la figure 1, celui-ci présente aussi une variante de montage en ce que les branchements à l'échangeur 1 des parties amont et aval 5, 16 et 6, 27 des premier et second circuits sont croisés, de sorte que les liquides s'écoulent à contre-courant dans les chambres 2, 3 de l'échangeur 1, ce qui est favorable aux transferts ioniques au travers de la membrane 3. Bien que ce montage à contre-courant augmente théoriquement les risques de rétrofiltration par rapport au montage à co-courant représenté sur la figure 1, toutes choses étant égales par ailleurs, dans le cas présent un tel risque est réduit : en effet, dans la mesure où d'une part, dans une installation d'hémodiafiltration on règle, de façon connue en soi, les pressions respectives dans le circuit sang et dans le circuit du liquide de dialyse de façon qu'il existe une pression transmembranaire positive entre la chambre baignée par le sang et la chambre baignée par le liquide de dialyse du dialyseur, et où, d'autre part, dans l'installation représentée sur la figure 2, la pression dans le circuit sang et la pression dans le circuit du liquide de substitution sont sensiblement égales, ces circuits étant en communication par la chambre de dégazage 28, il existe donc aussi, lorsque le dispositif d'hémodiafiltration fonctionne, une pression transmembranaire positive entre la chambre 2 baignée par le liquide de substitution et la chambre 3 baignée par le liquide de dialyse de l'échangeur 1.

La composition des premier et second liquides utilisés pour préparer le liquide de substitution dans ce second dispositif ne diffère pas de celle qui a été décrite plus haut. En revanche, la concentration des électrolytes qu'ils contiennent respectivement est ajustée pour que le liquide sortant de la deuxième chambre 3 de l'échangeur 1 ait les caractéristiques d'un liquide de dialyse. Cet ajustement des concentrations en fonction du taux de transfert dans l'échangeur 1 pour produire un liquide de substitution et un liquide de dialyse de composition et de concentration en électrolytes déterminées relève de l'application de lois connues sur les transferts ioniques au travers des membranes semi-perméables.

Dans ce second dispositif de préparation d'un liquide de substitution, le second liquide est avantageusement préparé en continu et réchauffé dans un générateur de liquide de dialyse 42, comprenant l'organe de chauffage 23, faisant partie du dispositif d'hémodiafiltration auquel le dispositif selon l'invention est associé. Pour que le liquide injecté au patient 12 ainsi que le second liquide, en aval de l'échangeur 1, qui sert de liquide de dialyse, soient à environ 37°C, l'organe de chauffage 23 porte le second liquide à une température supérieure à 37°C afin de permettre le réchauffage du premier liquide dans l'échangeur 1 et de compenser les pertes thermiques se produisant dans les premier et second circuits.

Le fonctionnement de ce dispositif ne diffère pas fondamentalement de celui qui a été décrit plus haut en référence au dispositif représenté sur la figure 1.

Il est à noter que, une fois que le circuit de premier liquide est connecté au réservoir 7 et que ce circuit est raccordé au circuit sang (canalisations 38, 41, chambre 35 du dialyseur 32) bar l'intermédiaire de la chambre de dégazage 28, le premier liquide peu servir de liquide de rinçage et de remplissage initial à la fois pour le circuit de premier liquide et pour le circuit sang.

La présente invention n'est pas limitée aux exemples de réalisation qui viennent d'être décrits et elle est susceptible de modifications et de variantes.

## Revendications

1. Procédé de préparation d'un liquide de substitution, caractérisé en ce qu'il consiste à faire s'écouler, de part et d'autre d'une membrane (4) semi-perméable d'un échangeur (1), un premier liquide stérile et apyrogène et un second liquide, ces liquides contenant au moins certains des électrolytes du sang dont au moins un agent tampon ou au moins un élément précurseur d'agent tampon, au moins certains de ces électrolytes ayant des concentrations différentes dans les deux liquides en amont de l'échangeur (1), le liquide de substitution étant constitué par le premier liquide en aval de l'échangeur (1).

2. Procédé selon la revendication 1, caractérisé en ce que l'un des liquides est chauffé en amont de l'échangeur (1) de façon que le premier liquide ait, en aval de l'échangeur (1), une température voisine de celle du corps humain.

3. Procédé selon une des revendications 1 et 2, caractérisé en ce que le second liquide est utilisé, en aval de l'échangeur (1), comme liquide de dialyse.

4. Procédé selon une des revendications 1 à 3, caractérisé en ce que les débits des liquides dans l'échangeur sont réglés pour que leur rapport soit sensiblement constant.

5. Procédé selon une des revendications 1 à 4, caractérisé en ce que le débit du second liquide est réglé en fonction lu débit du premier liquide.

6. Procédé selon une des revendications 1 à 5, caractérisé en ce que l'écoulement des liquides dans l'échangeur (1) se fait à contre-courant.

7. Procédé selon une des revendications 1 à 5, caractérisé en ce que l'écoulement des liquides dans l'échangeur (1) se fait à co-courant.

8. Procédé selon une des revendications 1 à 7, caractérisé en ce qu'une pression transmembranaire positive est maintenue entre la face de la membrane (4) baignée par le premier liquide et la face de la membrane (4) baignée par le second liquide.

9. Procédé selon une des revendications 1 à 8, caractérisé en ce que le premier liquide est utilisé comme liquide de rinçage et de remplissage initial pour un premier circuit destiné à joindre une source (7) de premier liquide à un patient.

10. Procédé selon une des revendications 1 à 9, caractérisé en ce que le premier liquide, en amont de l'échangeur (1), est une solution de chlorure de sodium.

11. Procédé selon une des revendications 1 à 9, caractérisé en ce que le premier liquide, en amont de l'échangeur (1), est une solution de chlorures de sodium, de calcium et de magnésium.

12. Procédé selon une des revendications 1 à 9, caractérisé en ce que le premier liquide contient, en amont de l'échangeur (1), du bicarbonate et pas de calcium ni de magnésium, et en ce que le second liquide contient, en amont de l'échangeur (1), du calcium et du magnésium et pas de bicarbonate.

13. Dispositif de préparation d'un liquide de substitution, caractérisé en ce qu'il comporte un échangeur (1) comprenant deux chambres (2, 3) séparées par une membrane semi-perméable (4), une première chambre (2) ayant une entrée (9) connectée à une source (7) d'un premier liquide stérile et apyrogène et une sortie (14) destinée à être connectée à un réceptacle (12), une seconde chambre (3) ayant une entrée (19) connectée à une source (17) d'un second liquide et une sortie (22) destinée à être connectée éventuellement à un dialyseur (32), ces liquides contenant au moins certains des électrolytes du sang dont au moins un agent tampon ou au moins un élément précurseur d'agent tampon, au moins certains de ces électrolytes ayant des concentrations différentes dans les deux liquides en amont de l'échangeur (1).

14. Dispositif selon la revendication 13, caractérisé en ce que la source de second liquide est un générateur de liquide de dialyse (42).

15. Dispositif selon une des revendications 13 et 14, caractérisé en ce qu'il comporte des moyens de chauffage (23) pour chauffer l'un des liquides en amont de l'échangeur (1).

16. Dispositif selon une des revendications 13 à 15, caractérisé en ce qu'il comporte une première pompe (10) pour faire s'écouler le premier liquide et une seconde pompe (20, 34) pour faire s'écouler le second liquide.

17. Dispositif selon la revendication 16, caractérisé en ce qu'il comporte des moyens de commande (25) pour régler le débit de la seconde pompe (20) en fonction du débit de la première pompe (10).

18. Dispositif selon la revendication 17, caractérisé en ce qu'il comporte un débitmètre (11) pour mesurer le débit du premier liquide et en ce que les moyens de commande (25) règlent le débit de la première pompe (10) en fonction de la comparaison entre la mesure fournie par le débitmètre (11) et au moins une valeur de consigne.

19. Dispositif selon une des revendications 13 à 18, caractérisé en ce que la sortie (14) de la première chambre (2) de l'échangeur (1) est connectée à un filtre bactérien (15).

## Patentansprüche

1. Verfahren zur Herstellung einer Substitutionsflüssigkeit, dadurch gekennzeichnet, daß man eine sterile und pyrogenfreie erste Flüssigkeit und eine zweite Flüssigkeit auf beiden Seiten einer semipermeablen Membran (4) eines Austauschers (1) fliessen lässt, wobei die Flüssigkeiten wenigstens bestimmte Elektrolyten des Blutes enthalten, welche wenigstens ein Pufferagens oder wenigstens ein Vorläuferelement eines Pufferagens enthalten, wobei wenigstens bestimmte dieser Elektrolyten unterschiedliche Konzentrationen in den zwei Flüssigkeiten stromaufwärts des Austauschers (1) besitzen, wobei die Substitutionsflüssigkeit aus der ersten Flüssigkeit stromabwärts des Austauschers (1) besteht.

2. Verfahren nach Anspruch 1, dadurch gekennzeichnet, daß eine der Flüssigkeiten stromaufwärts des Austauschers (1) erhitzt wird, so daß die erste Flüssigkeit stromabwärts des Austauschers (1) eine dem menschlichen Körper ähnliche Temperatur aufweist.

3. Verfahren nach einem der Ansprüche 1 und 2, dadurch gekennzeichnet, daß die zweite Flüssigkeit stromabwärts des Austauschers (1) als Dialyseflüssigkeit verwendet wird.

4. Verfahren nach einem der Ansprüche 1 bis 3, dadurch gekennzeichnet, daß die Durchsätze der Flüssigkeiten durch den Austauscher geregelt werden, damit ihr Verhältnis etwa konstant ist.

5. Verfahren nach einem der Ansprüche 1 bis 4, dadurch gekennzeichnet, daß der Durchsatz der zweiten Flüssigkeit in Abhängigkeit von dem Durchsatz der ersten Flüssigkeit geregelt wird.

6. Verfahren nach einem der Ansprüche 1 bis 5, dadurch gekennzeichnet, daß das Fliessen der Flüssigkeiten in den Austauscher (1) im Gegenstrom erfolgt.

7. Verfahren nach einem der Ansprüche 1 bis 5, dadurch gegennzeichnet, daß das Fliessen der Flüssigkeiten in den Austauscher (1) im Gleichstrom erfolgt.

8. Verfahren nach einem der Ansprüche 1 bis 7, dadurch gekennzeichnet, daß ein positiver transmembraner Druck zwischen der Seite der Membran (4), welche durch die erste Flüssigkeit benetzt wird, und der Seite der Membran (4), welche durch die zweite Flüssigkeit benetzt wird, aufrecht erhalten wird.

9. Verfahren nach einem der Ansprüche 1 bis 8, dadurch gekennzeichnet, daß die erste Flüssigkeit als Spülflüssigkeit und als Anfangsfüllflüssigkeit für einen ersten Kreislauf, welcher dazu bestimmt ist, eine Quelle (7) der ersten Flüssigkeit mit dem Patienten zu verbinden, verwendet wird.

10. Verfahren nach einem der Ansprüche 1 bis 9, dadurch gekennzeichnet, daß die erste Flüssigkeit stromaufwärts des Austauschers (1) eine Natriumchlorid-Lösung ist.

11. Verfahren nach einem der Ansprüche 1 bis 9, dadurch gekennzeichnet, daß die erste Flüssigkeit stromaufwärts des Austauschers (1) eine Lösung der Chloride von Natrium, Kalzium und Magnesium ist.

12. Verfahren nach einem der Ansprüche 1 bis 9, dadurch gekennzeichnet, daß die erste Flüssigkeit stromaufwärts des Austauschers (1) Bikarbonat und weder Kalzium noch Magnesium enthält und daß die zweite Flüssigkeit stromaufwärts des Austauschers (1) Kalzium und Magnesium und kein Bikarbonat enthält.

13. Vorrichtung zur Herstellung einer Substitutionsflüssigkeit, dadurch gekennzeichnet, daß sie einen Austauscher (1) enthält, welcher zwei Kammern (2,3) umfaßt, welche durch eine semipermeable Membran (4) getrennt sind, wobei eine erste Kammer (2) einen Einlaß (9), welcher mit einer Quelle (7) für eine erste sterile und pyrogen-freie Flüssigkeit verbunden ist, und einen Auslaß (14), welcher für eine Verbindung an einen Aufnahmebehälter (12) bestimmt ist, aufweist und eine zweite Kammer (3) einen Einlaß (19), welcher mit einer Quelle (17) für eine zweite Flüssigkeit verbunden ist, und einen Auslaß (22), welcher dazu bestimmt ist, gegebenenfalls mit einem Dialysator (32) verbunden zu werden, aufweist, wobei diese Flüssigkeiten wenigstens bestimmte Elektrolyten des Blutes enthalten, welche wenigstens ein Pufferagens oder wenigstens ein Vorläuferelement eines Pufferagens enthalten, wobei wenigstens bestimmte dieser Elektrolyten unterschiedliche Konzentrationen in den zwei Flüssigkeiten stromaufwärts des Austauschers (1) besitzen.

14. Vorrichtung nach Anspruch 13, dadurch gekennzeichnet, daß die Quelle der zweiten Flüssigkeit ein Dialyseflüssigkeits-Erzeuger (42) ist.

15. Vorrichtung nach einem der Ansprüche 13 und 14, dadurch gekennzeichnet, daß sie Heizeinrichtungen (23) zum Erhitzen der einen der stromaufwärts des Austauschers (1) befindlichen Flüssigkeiten enthält.

16. Vorrichtung nach einem der Ansprüche 13 bis 15, dadurch gekennzeichnet, daß sie eine erste Pumpe (10) zu die erste Flüssigkeit fliessen lassen und eine zweite Pumpe (20,34) zu die zweite Flüssigkeit fliessen lassen, umfaßt.

17. Vorrichtung nach Anspruch 16, dadurch gekennzeichnet, daß sie Steuereinrichtungen (25) zum Regeln des Durchsatzes der zweiten Pumpe (20) in Abhängigkeit von dem Durchsatz der ersten Pumpe (10) umfaßt.

18. Vorrichtung nach Anspruch 17, dadurch gekennzeichnet, daß sie ein Mengenmeßgerät (11) zum Messen des Durchsatzes der ersten Flüssigkeit umfaßt, und daß sie Steuereinrichtungen (25) umfaßt, welche den Durchsatz der ersten Pumpe (10) als Funktion des Vergleichs zwischen der Messung, welche von dem Mengenmeßgerät (11) geliefert wird, und wenigstens einem Vergleichswert regelt.

19. Vorrichtung nach einem der Ansprüche 13 bis 18, dadurch gekennzeichnet, daß der Ausgang (14) der ersten Kammer (2) des Austauschers (1) mit einem Bakterienfilter (15) verbunden ist.

## Claims

1. A method for preparing a substitution liquid, the method being characterized in that it consists in causing a sterile and pyrogen-free first liquid and a second liquid to flow over opposite sides of a semipermeable membrane (4) in an exchanger (1), said liquids containing at least some of the electrolytes of blood including at least one buffer agent or at least one precursor of a buffer agent, at least some of the electrolytes being at different concentrations in the two liquids upstream from the exchanger (1), the substitution liquid being constituted by the first liquid downstream from the exchanger (1).

2. A method according to claim 1, characterized in that one of the liquids is heated upstream from the exchanger (1) so that downstream from the exchanger (1) the first liquid has a temperature close to that of the human body.

3. A method according to claim 1 or 2, characterized in that the second liquid is used downstream from the exchanger (1) as a dialysis liquid.

4. A method according to any one of claims 1 to 3, characterized in that the liquid flow rates through the exchanger are adjusted so that their ratio is substantially constant.

5. A method according to any one of claims 1 to 4, characterized in that the flow rate of the second liquid is adjusted as a function of the flow rate of the first liquid.

6. A method according to any one of claims 1 to 5, characterized in that the liquids flow through the exchanger (1) in a counterflow disposition.

7. A method according to any one of claims 1 to 5, characterized in that the liquids flow through the exchanger (1) in a same-direction parallel-flow disposition.

8. A method according to any one of claims 1 to 7, characterized in that a positive transmembrane pressure is maintained between the face of the membrane (4) in contact with the first liquid and the face of the membrane (4) in contact with the second liquid.

9. A method according to any one of claims 1 to 8, characterized in that the first liquid is used as a washing liquid and as a priming liquid for a first circuit connecting a source (7) of the first liquid to a patient (12).

10. A method according to any one of claims 1 to 9, characterized in that upstream from the exchanger (1) the first liquid is a solution of sodium chloride.

11. A method according to any one of claims 1 to 9, characterized in that upstream from the exchanger (1) the first liquid is a solution of sodium, calcium, and magnesium chlorides.

12. A method according to any one of claims 1 to 9, characterized in that upstream from the exchanger (1) the first liquid contains bicarbonate and no calcium or magnesium, and in that, upstream from the exchanger (1), the second liquid contains calcium and magnesium, but no bicarbonate.

13. Apparatus for preparing a substitution liquid, the apparatus being characterized in that it comprises an exchanger (1) comprising two chambers (2, 3) separated by a semipermeable membrane (4), a first chamber (2) having an inlet (9) connected to a source (7) of a first liquid which is sterile and pyrogen-free, and an outlet (14) for connection to a receptacle (12), a second chamber (3) having an inlet (19) connected to a source (17) of a second liquid, and an outlet (22) for optional connection to a dialyzer (32), said liquids containing at least some of the electrolytes of blood and including at least one buffer agent or precursor for a buffer agent, at least some of the electrolytes being at different concentrations in the two liquids upstream from the exchanger (1).

14. Apparatus according to claim 13, characterized in that the source of the second liquid is a dialysis liquid generator (42).

15. Apparatus according to claim 13 or 14, characterized in that it includes heater means (23) for heating one of the liquid upstream from the exchanger (1).

16. Apparatus according to any one of claims 13 to 15, characterized in that it includes a first pump (10) for causing the first liquid to flow, and a second pump (20, 34) for causing the second liquid to flow.

17. Apparatus according to claim 16, characterized in that it includes control means (25) for adjusting the flow rate of the second pump (20) as a function of the flow rate of the first pump (10).

18. Apparatus according to claim 17, characterized in that it includes a flow meter (11) for measuring the flow rate of the first liquid, and in that the control means (25) adjust the flow rate of the first pump (10) as a function of a comparison between the measurements provided by the flow meter (11) and at least one reference value.

19. Apparatus according to any one of claims 13 to 18, characterized in that the outlet (14) of the first chamber (2) of the exchanger (1) is connected to a bacteria filter (15).
